# EUROPEAN PATENT SPECIFICATION

(11) **EP 4 279 099 B1**
(45) Date of publication and mention of the grant of the patent: **13.05.2026**
(21) Application number: 21919049.3
(22) Date of filing: 02.12.2021
(51) Int. Cl.: A61M 5/142, A61M 5/36, A61M 39/28

(54) **INFUSION DEVICE**
INFUSIONSVORRICHTUNG
DISPOSITIF DE PERFUSION

(30) Priority: 13.01.2021 CN 202120085804 U
(43) Date of publication of application: 22.11.2023
(73) Proprietor: Fresenius Kabi (Nanchang) Co., Ltd., Nanchang, Jiangxi 330013 (CN)
(72) Inventor: HU, Qing, Nanchang, Jiangxi 330013 (CN); ZOU, Zhenzhong, Nanchang, Jiangxi 330013 (CN); XU, Jian, Nanchang, Jiangxi 330013 (CN); LAU, Fat Kit, Nanchang, Jiangxi 330013 (CN)
(74) Representative: Fresenius Kabi Deutschland GmbH
(86) International application number: PCT/CN2021/134974
(87) International publication number: WO 2022/151858

(56) References cited:
- WO-A1-2014/030184
- WO-A1-2020/153036
- CN-A- 101 795 724
- CN-A- 101 795 724
- CN-A- 110 234 376
- JP-A- 2012 029 914
- JP-A- H0 992 243
- US-A1- 2002 019 612
- US-A1- 2002 019 612
- US-A1- 2017 165 414
- US-A1- 2019 117 878

## Description

### TECHNICAL FIELD

The present application relates to infusion devices for delivering medical fluids such as drugs or nutrient solutions to patients.

### BACKGROUND

Infusion devices, such as volumetric (peristaltic) infusion devices, are used to accurately deliver medical fluids such as drugs or nutrient solutions to patients at a predetermined rate. An infusion line passing through the infusion device is connected at one end to, for example, a container containing liquid medicine, and at the other end to a patient, where a pumping mechanism in the infusion device exerts a pumping force on the infusion line, for example, in a peristaltic manner, so that the liquid medicine is transported toward the patient through the infusion line.

Generally, in order to better fix the infusion line in the infusion device, a buckle part for fixing the infusion line in place is provided in the infusion device. However, on the one hand, when the buckle of the buckle part to the infusion line is loose, the infusion line may not be well fixed in the infusion device and the infusion line may not be snugly buckled between the bubble sensors, resulting in false alarm caused by the gap between the bubble sensors. On the other hand, when the buckle part is tightly buckled to the infusion line, although the infusion line may be snugly buckled between the bubble sensors, in this case, the buckle part is easy to block the infusion line, thus affecting the infusion operation.

In addition, when arranging the infusion line on the infusion device, it should be avoided that the medical liquid flows through the infusion line in an uncontrolled manner, the so-called free flow state. To this end, the infusion device includes a liquid stopper mechanism that stops the flow through the infusion line before starting the actual infusion operation, so as to be able to perform, for example, an integrity check of the infusion device, especially a so-called OCS (Obstructive Inspection System) test, etc. At the beginning of the infusion operation, the liquid stopper mechanism is opened so that the flow through the infusion line may take place under the control of the pumping mechanism.

However, in multiple operations, the traditional liquid stopper mechanism is prone to component wear during the opening and closing process, resulting in deterioration. In addition, when the liquid stopper mechanism is not accurately positioned, it may be difficult for the liquid stopper mechanism to clamp the infusion line, thus causing the medical liquid to flow through the infusion device in an undesired manner.

In addition, the traditional infusion device has more connecting parts, which increases the production and assembly cost of the infusion device.

Therefore, it is desired to realize a new type of infusion device with an improved design, which may at least eliminate or improve some or all of the above-mentioned problems. An infusion device illustrating the state of the art is known from US 2019/117878 A1.

It should be noted that the background technology section is intended to illustrate the technical background of the present application, and is not intended to limit the scope of the present application. It should be noted herein that the technical content provided in this section is intended to help those skilled in the art to understand the present application, and may not necessarily constitute the prior art.

### SUMMARY

The invention comprises an infusion device according to claim 1. Additional features of preferred embodiments of the invention are defined in the dependent claims. A general summary of the present application is provided in this section, rather than the full scope of the present application or a comprehensive disclosure of all features of the present application.

An object of the present disclosure is to provide a new type of infusion device, which enables the infusion line be well fixed in place in the infusion device without causing blockage of the infusion line.

Another object of the present disclosure is to provide a new type of infusion device, which enables the bubble sensor of the infusion device more accurately detect the bubbles in the infusion line.

Another object of the present disclosure is to provide a new type of infusion device whose liquid stopper mechanism is smoother and does not wear out during the opening and closing process.

Another object of the present disclosure is to provide a new type of infusion device, which may ensure the accurate positioning of the liquid stopper mechanism.

Another object of the present disclosure is to provide a new type of infusion device with a speaker assembly that is easy to mount.

According to an aspect of the present disclosure, there is provided an infusion device, where the infusion device includes a body, the body is configured to receive an infusion line and include a pumping mechanism, the pumping mechanism is configured to enable medical fluid to flow through the infusion line received in the body; a pump door assembly, the pump door assembly is configured to be connected to the body for closing the body, and is configured to fix the infusion line in place in the body, the pump door assembly includes a buckle part. The buckle part includes: buckle protrusions provided at both ends of the buckle part along the longitudinal direction of the buckle part, and top surfaces of the buckle protrusions are shaped to be suitable for receiving the infusion line; a groove, the groove being located at an intermediate position between the buckle protrusions and configured to be recessed in the height direction of the buckle part to be lower than the buckle protrusions.

In the above infusion device, the buckle part further includes transition recesses configured to extend between the groove and the buckle protrusion from both sides of both longitudinal ends of the groove.

In the above infusion device, the body of the infusion device further includes bubble sensors, the bubble sensors are arranged at both sides of the infusion line received in the body.

In the above infusion device, the distance between the bubble sensors is smaller than the diameter of the infusion line.

In the above infusion device, the body includes a liquid stopper mechanism, the liquid stopper mechanism includes: a clamping valve which may be deformed between a clamping position and a release position, in the clamping position, the clamping valve presses the infusion line passing through the clamping valve to block the flow of medical fluid in the infusion line, in the release position, the clamping valve allows the medical fluid to flow through the infusion line, where the contour of the upper surface of the clamping valve is a smoothly curved curve; a lever element, the level element may be operated to act on the upper surface of the clamping valve for switching the clamping valve between the clamping position and the release position.

In the above infusion device, the liquid stopper mechanism includes an infrared recognition assembly, and the infrared recognition assembly includes: an infrared transmitter; an infrared receiver; and an infrared light guide, the infrared light guide includes: two light guide posts, the two light guide posts are respectively positioned directly above the infrared transmitter and the infrared receiver; and a connecting part, the connecting part connects the two light guide posts.

In the above infusion device, the connecting part further includes an anti-light-guiding structure, and the anti-light-guiding structure is configured as two grooves formed on two opposite surfaces of the connecting part, and the grooves extend along the width direction of the connecting part and are staggered with each other, so that the side profile of the anti-light-guiding structure is S-shaped.

In the above infusion device, the infrared light guide is a single-piece component formed by integral molding.

In the above infusion device, the body further includes a fixing piece for receiving the infusion line, and the fixing piece includes elastic legs at two sides of the fixing piece, and when the infusion line is received in the fixing piece, the elastic legs are elastically deformed so that the infusion line is clamped in the fixing piece.

In the above infusion device, the bottom part of the fixing piece is configured to have a shape structure matching with the corresponding part of the body for connecting the fixing piece, and the fixing piece is connected to the body in a hot melt connection manner.

In the above infusion device, the infusion device further includes a loudspeaker assembly, the loudspeaker assembly includes: a battery door cover, the battery door cover is located at the top of the loudspeaker assembly, and the battery door cover is provided with two connecting protrusions protruding and extending at one side of the battery door cover; loudspeaker; a battery door base, the battery door base is configured to receive the battery door cover and the loudspeaker, and the battery door base is configured to have a receiving recess for receiving the connecting protrusion; and a connecting piece, the connecting piece extends through the battery door cover and the battery door base to fixedly connect the battery door cover and the battery door base.

In one embodiment of the present disclosure, the buckle part of the pump door assembly is configured to have a groove adapted to receive an infusion line. With this design, the infusion line will be well fixed in place in the infusion device without causing blockage of the infusion line.

Preferably, the buckle part further includes transition recesses for further accommodating the infusion line received in the recess.

Preferably, bubble sensors arranged on both sides of the infusion line in the infusion device can detect bubbles in the infusion line, and trigger an alarm to remind the operator to check when bubbles are detected, so as to prevent the gas from inputting into the patient and causing damage to the patient.

Preferably, the distance between the bubble sensors is smaller than the diameter of the infusion line. With the help of this size design and the above buckle parts, the infusion line can be tightly filled between the bubble sensors, so that there is no gap between the bubble sensors due to the lack of tight filling, thus preventing the bubble sensors from detecting the air existing due to the gap and causing false alarms.

On the other hand, preferably, the infusion device according to the present disclosure further includes a clamping valve having a novel contoured surface. With this design, the user can open and close the clamping valve more smoothly and reduce the wear on the clamping valve and other components.

Besides, preferably, with the aid of the above infrared recognition assembly, the operator can easily confirm whether the clamping valve is accurately positioned in the infusion device. With the aid of the above anti-light-guiding structure, the propagation of light in the connecting part can be reduced, thereby improving the recognition accuracy of the infrared recognition assembly. Therefore, it is possible to form the infrared light guide as a single-piece component in, for example, an integrated manner while allowing light to propagate only through the light guide column of the infrared light guide. This reduces the production cost and manufacturing difficulty of the infrared light guide.

Furthermore, it is advantageous that the elastic legs of the above fixing piece enable the operator to easily confirm that the infusion line is completely received in the fixing piece. In addition, since the fixing piece is configured to be hot-melt connected with the body, this improves the waterproof performance of the infusion device and reduces the number of connecting parts in the infusion device.

In addition, the above loudspeaker assembly is assembled by two connecting protrusions and a connecting piece. With this connection design, the number of connecting parts in the infusion device is further reduced, thereby reducing the manufacturing cost of the infusion device and simplifying the assembly process of the infusion device.

### BRIEF DESCRIPTION OF THE DRAWINGS

Features and advantages of one or more embodiments of the present application will become easier to understand through the following description in conjunction with the drawings. It should be understood that the drawings are only shown in a schematic manner, and the embodiments of the present application are not limited to the examples shown in the drawings. For clarity, same or similar components in the drawings are indicated with same reference numerals. In the drawings,
Figure 1 schematically shows a view of the infusion device according to the present application in a working state;
Figure 2 schematically shows a perspective view of a pump door assembly of an infusion device according to the present application and a partial enlarged view of a buckle part included therein;
Figure 3 schematically shows a top view of the body of the infusion device according to the present application;
Figure 4 is a partial sectional view taken along line A-A in Figure 3;
Figure 5 schematically shows a cross-sectional view of an infusion device according to the present application, in which a buckle part of a pump door assembly has just started to contact an infusion line received in a body;
Figures 6A to 6C schematically show a process of pressing an infusion line into a target position by a buckle part in a partial sectional view;
Figure 7 schematically shows a cross-sectional view of an infusion device according to the present application when an infusion line is pressed to a target position by a buckle part;
Figures 8A and 8B show the clamping valve in a release position and a clamping position;
Figure 9 schematically shows the infrared recognition assembly in an assembled state in a partial cross-sectional view;
Figure 10 schematically shows a perspective view of an infrared light guide;
Figures 11A and 11B schematically show the fixing piece and the body of the infusion device in a separated and connected state;
Figures 12A to 12C schematically illustrate a process of receiving an infusion line by a fixing piece;
Figure 13A schematically show an exploded perspective view of a loudspeaker assembly;
Figure 13B schematically shows a partial cross-sectional view of the loudspeaker assembly in an assembled state.

### DETAILED DESCRIPTION

The present application is described in detail hereinafter by means of exemplary embodiments with reference to the accompanying drawings. It should be understood that the following detailed description of the present application is only intended for illustration, rather than limiting the present application and its applications or uses.

Orientation terms such as "upper", "lower", "left", "right" and the like used in the description are only intended for clear description in conjunction with the drawings, rather than limiting the orientation of related components. In actual operation, the position and orientation relationship among components can be changed according to specific applications.

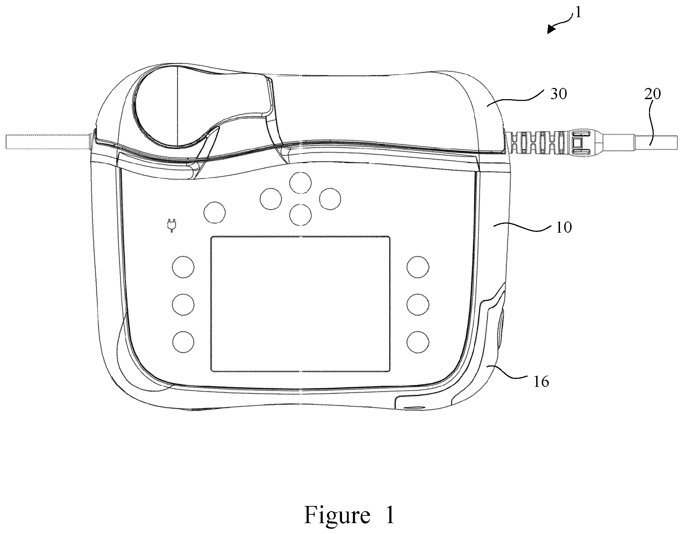

Figure 1 schematically shows a view of the infusion device 1 according to an embodiment of the present application in a working state. As shown in Figure 1, the infusion device 1 includes: a body 10, the body 10 is configured to receive internal components of the infusion device and receive an infusion line 20 passing through the infusion device; a pump door assembly 30, the pump door assembly 30 is pivotally connected to a top portion of the body 10 and closes the top of the body 10 in an operating state; and the loudspeaker assembly 16, the loudspeaker assembly is mounted on the side of the body 10 for alarming or prompting the operator of the infusion device 1. In addition, the body 10 also contains power supply components, a pumping mechanism, electronic components for controlling the flow of medical fluid, and various sensors for detecting the flow state of the medical fluid. The surface of the body 10 is provided with components such as buttons and displays for the operator to input and observe the preset parameters of the medical fluid infusion. During the operation of the infusion device 1, the operator inputs preset infusion parameters through buttons on the surface of the body 10, and then the electronic components in the body 10 control the pumping mechanism to exert a pumping effect on the infusion line 20 received in the body 10 in a controllable manner, so that the medical fluid in the infusion line 20 flows to the patient in a desired flow state.

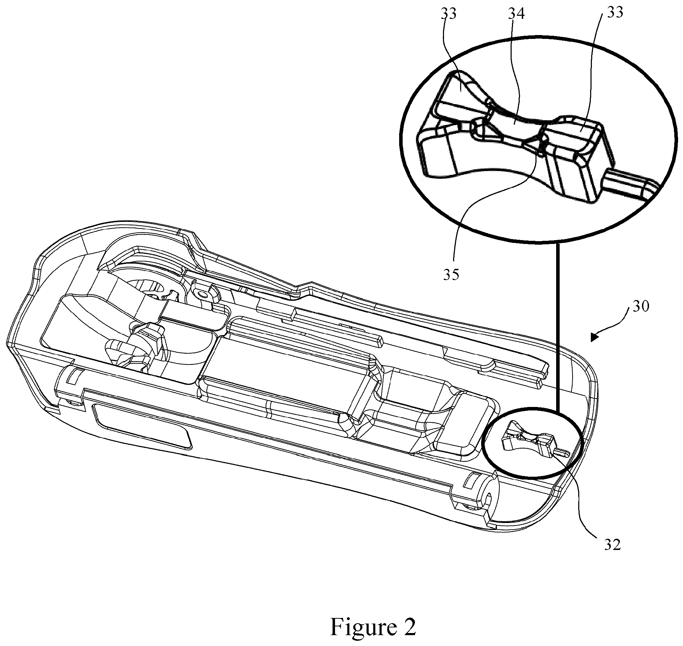

Figure 2 schematically shows a perspective view of the pump door assembly 30 and a partial enlarged view of the buckle part 32 included in the pump door assembly 30. As shown in Figure 2, the pump door assembly 30 includes a buckle part 32 formed on the inner side of the pump door assembly, which is used to fix the infusion line 20 in place in the body 10 when the pump door assembly 30 closes the body 10. As shown in a partial enlarged view in Figure 2, the buckle part 32 includes: buckle protrusions 33 provided at both ends of the buckle part 32 in the longitudinal direction thereof, and the top surfaces of the buckle protrusions 33 are shaped to receive the infusion line 20, for example, formed with recesses adapted to receive the infusion line 20; a groove 34, the groove 34 is located at an intermediate position between the buckle protrusions 33 and configured to be recessed in a substantially arc shape in the height direction of the buckle part 32 to be lower than the buckle protrusions 33; and transition recesses 35, the transition recesses 35 are configured to extend between the groove 34 and the buckle protrusions 33 on both sides of both longitudinal ends of the groove 34.

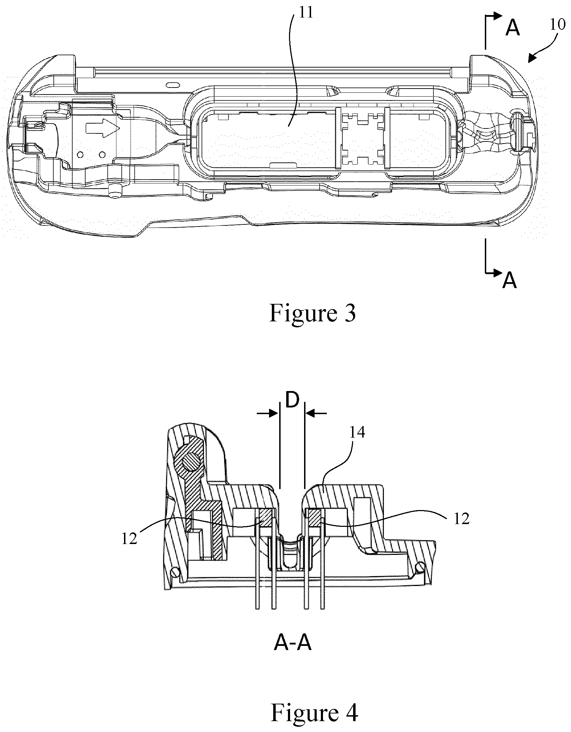

Figure 3 schematically shows a top view of the top of the body of the infusion device according to the present application. As shown in Figure 3, the body 10 includes a pumping mechanism 11, which exerts a pumping force on the infusion line in a manner known per se, so that the liquid medicine is delivered to the patient through the infusion line.

Figure 4 is a partial cross-sectional view of the body 10 taken along the line A-A in Figure 3. As shown in Figure 4, the body 10 further includes two receiving parts 14, the receiving parts are configured to cooperate with the buckle parts 32 of the pump door assembly 30 to receive the infusion line 20 mounted in the body 10 and limit the infusion line 20. Preferably, the tops of the receiving parts 14 have a chamfered configuration to facilitate the infusion line to be easily received between the receiving parts 14; and a bubble sensor 12 arranged directly below the receiving part 14 and aligned with the receiving part 14 in the lateral direction. The lateral distance D between the bubble sensors 12 is smaller than the outer diameter of the infusion line 20.

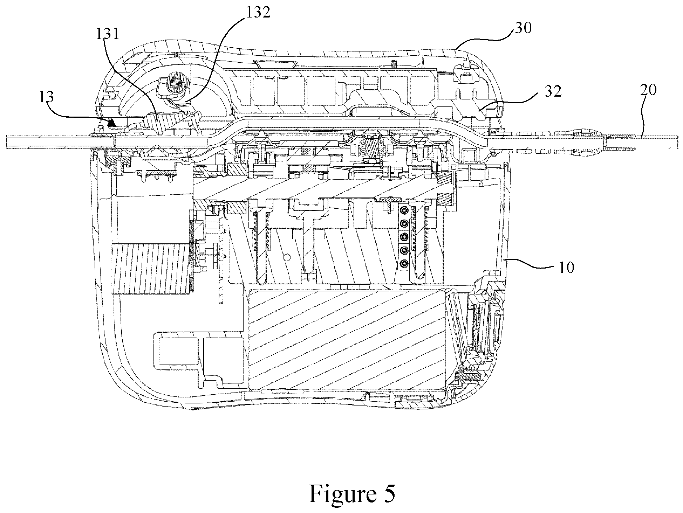

Figure 5 schematically shows a cross-sectional view of the infusion device when the buckle part just comes into contact with the infusion line received in the body. Since the infusion line 20 is flexible and the distance between the receiving parts 14 is smaller than the diameter of the infusion line 20, certain upward camber deformation occurs when the infusion line 20 is received between the receiving parts 14 in the body 10 to enter the area where the bubble sensor 12 is located. Since the distance between the receiving parts 14 is smaller than the outer diameter of the infusion line 20, during the process of receiving the infusion line 20 between the receiving parts 14, the outer wall of the infusion line 20 and the side wall of the receiving parts 14, that is, the side wall where the bubble sensors 12 are located, will generate a large friction force and may generate a deformed part of the infusion line 20 and cause the infusion line 20 not to be in the center position between the bubble sensors.

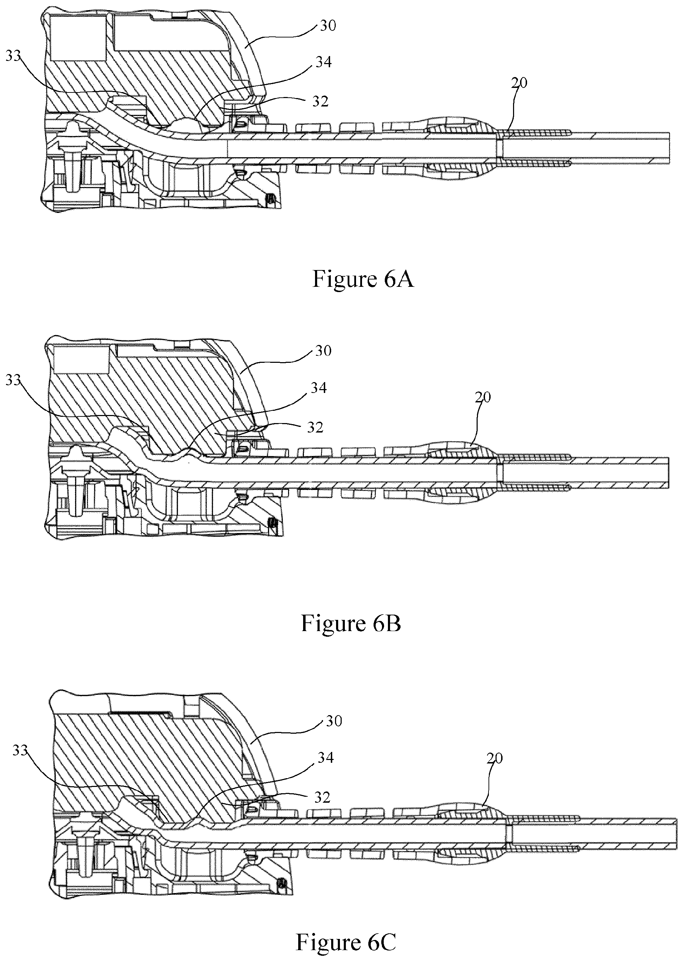

In the state of Figure 5, the buckle part 32 of the pump door assembly 30 has just contacted the outer wall of the infusion line 20 and has not yet acted on the infusion line 20. Hereinafter, the process of pressing the infusion line 20 into the target position by the buckle part 32 is described with reference to Figures 6A to 6C. In the process of closing the pump door assembly 30 downward, the buckle protrusions 33 in the buckle part 32 first comes into contact with the infusion line 20 and presses down the infusion line 20 so that the infusion line 20 may be received in the receiving part 14 as described above. Since the infusion line 20 itself has flexibility, during the process that the infusion line 20 is pressed down by the buckle protrusions 33, the upward arched deformation generated by the infusion line 20 is accommodated in the groove 34 of the buckle part 32. In the process that the buckle protrusions 33 gradually press down the infusion line 20, the groove 34 accommodates the continuously generated deformation part of the infusion line 20, thereby reducing the resistance in the process that the infusion line 20 is pressed down to the target position, so that the buckle part 32 may press down the infusion line 20 to the target position without blocking the infusion line 20. Particularly, when the deformed part of the infusion line 20 is too large, the deformed part accommodated in the groove 34 may be further deformed to be accommodated in the transition recesses 35 (not shown in the Figure).

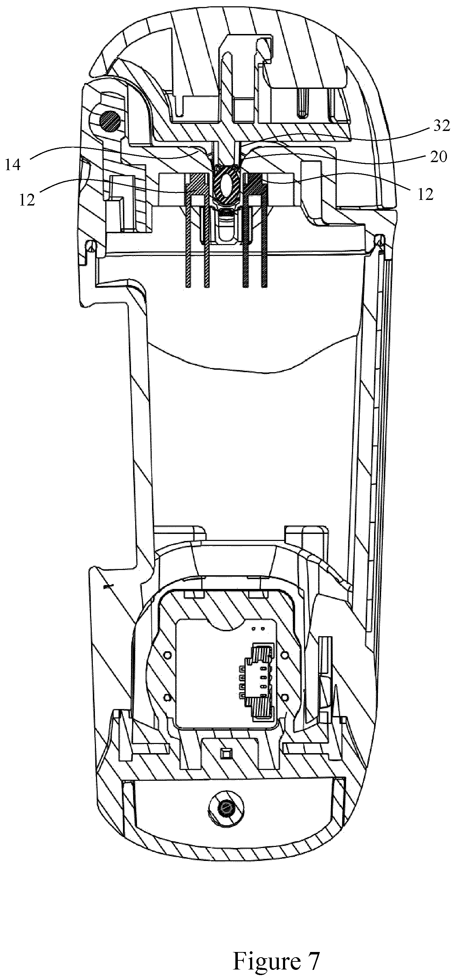

Figure 7 schematically shows a cross-sectional view of the infusion device 1 when the infusion line 20 is pressed to the target position by the buckle part 32. As shown in Figure 7, in this state, the infusion line 20 is closely attached between the bubble sensors 12, so that it is possible to prevent the bubble sensors 12 from causing false alarms due to gaps between the bubble sensors 12.

Referring back to Figure 5, the body 10 further includes a liquid stopper mechanism 13. The liquid stopper mechanism 13 is provided at the other end of the body 10 opposite to the buckle part 32, and is used for restraining the infusion line 20 passing through the infusion device 10 in a proper position together with the buckle part 32. At the same time, the liquid stopper mechanism 13 is also used to turn on and off the fluid flowing through the infusion line 20. Specifically, the liquid stopper mechanism 13 includes a clamping valve 131 and a lever element 132, where the lever element 132 acts on the upper surface of the clamping valve 131, so that the clamping valve 131 deforms between a clamping position and a release position with the rotation of the lever element 132. Therefore, on one hand, the liquid stopper mechanism 13 may limit the infusion line 20 received therein, and on the other hand, before starting the actual infusion operation, for example, when the integrity of the infusion device needs to be checked and tested, the liquid stopper mechanism 13 is in its clamping position to clamp the infusion line 20 so as to prevent the medical fluid from flowing through the infusion line 20 in an uncontrolled manner. When the infusion operation is about to start, the liquid stopper mechanism 13 is in its release position to allow medical fluid to flow through the infusion line 20.

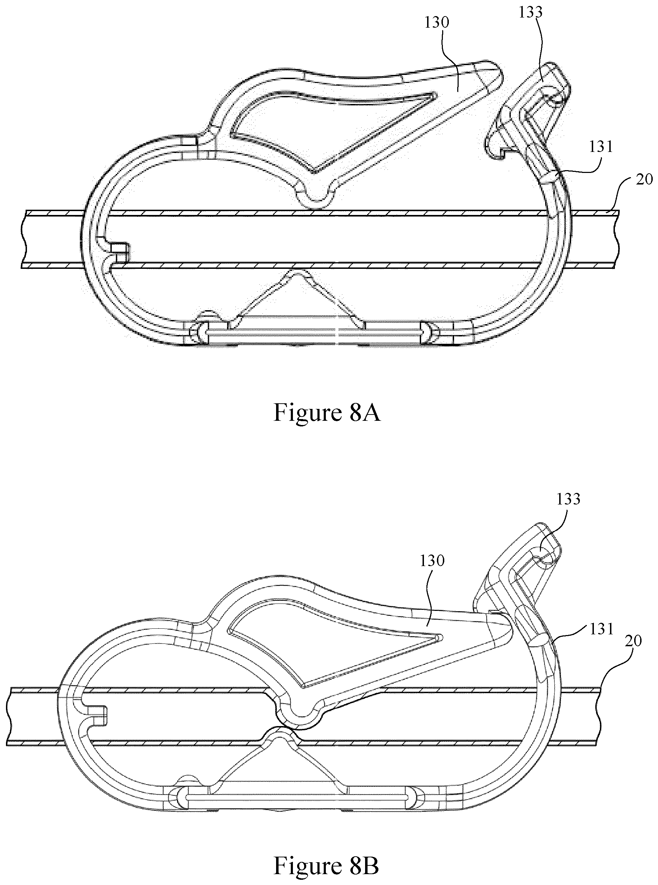

Figures 8A and 8B show the clamping valve 131 in the release position and the clamping position. as shown in Figures. 8A and 8B, the contour of the upper surface of the clamping valve 131 is shaped to have a smoothly curved curve. An operator drives the lever element 132 to rotate through a wrench arranged outside the body, and the lever element 132 is in sliding fit with the upper surface of the clamping valve 131, so that the free end 130 of the clamping valve 131 is driven to move downwards to clamp the infusion line 20 and is clamped into the clamping position shown in Figure 8B by the stopper 133. The clamping valve 131 may compress the infusion line 20 to block the flow of medical fluid in the infusion line 20. When opening the clamping valve 131, the operator may, for example, toggle the stopper 133 through a member provided outside the body, and rotate a wrench to drive the lever element 132 to rotate in the opposite direction, thereby bringing the clamping valve 131 into a release position as shown in Figure 8A, where the clamping valve 131 allows the flow of medical fluid through the infusion line 20. Since the upper surface of the clamping valve 131 has a smooth curved design, the sliding contact between the lever element 132 and the upper surface of the clamping valve can be carried out in a smoother manner during the opening and closing process of the clamping valve 131, and the deterioration of components caused by the wear of the clamping valve can be reduced.

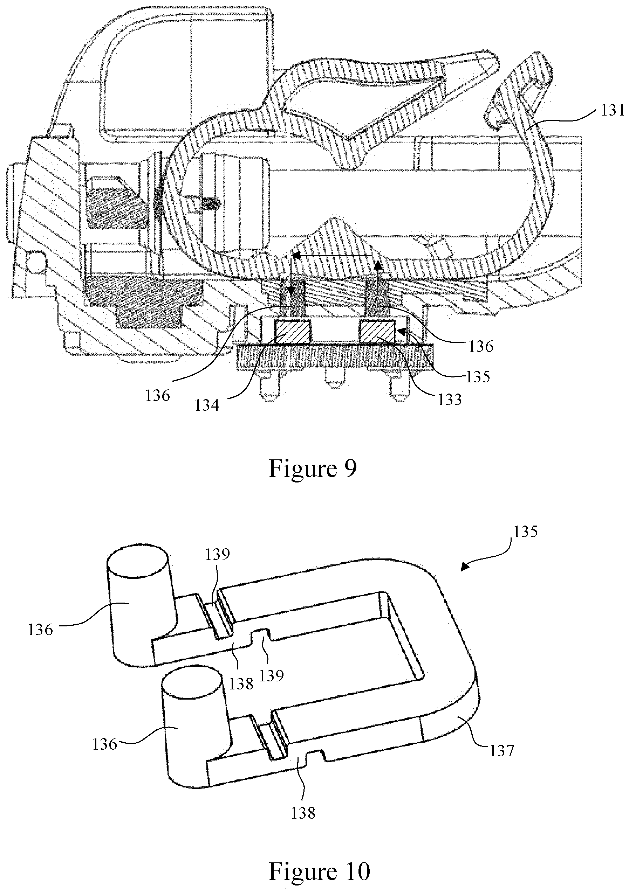

In order to facilitate the operator to confirm that the liquid stopper mechanism 13 is correctly positioned in the body 10, the liquid stopper mechanism 13 further includes an infrared recognition assembly, which is mounted below the clamping valve 131. Figure 9 schematically shows the infrared recognition assembly in an assembled state in a partial cross-sectional view. The infrared recognition assembly includes an infrared transmitter 133, an infrared receiver 134 and an infrared light guide 135. As shown in Figure 9, the infrared transmitter 133 emits infrared light upward, and the infrared light passes through the two light guide columns 136 of the infrared light guide 135 and the bottom part of the clamping valve in the light path direction indicated by the arrow in the Figure to reach the infrared receiver 134. Through the infrared signal received by the infrared receiver 134, the operator can easily confirm whether the clamping valve 131 is correctly positioned in the body 10.

Figure 10 schematically shows a perspective view of the infrared light guide 135. As shown in Figure 10, the infrared light guide 135 includes two light guide column 136 and a connecting part 137 connecting the two light guiding posts, where an anti-light-guiding structure 138 is arranged near two ends of the connecting part 137 adjacent to the light guiding column 136, thereby preventing and reducing the transmission loss of infrared light along the connecting part 137 and improving the light guiding amount of the light guide 135 through the two light guiding columns 136. In the embodiment of Figure 10, each anti-light-guiding structure 138 is configured to have two grooves 139, which are formed at positions where the top surface and the bottom surface of the connecting part 137 are staggered and extend over the entire width of the connecting part 137. The depth of each groove 132 may be half to 2/3 of the thickness of the connecting part 137, so that the side profile of the anti-light-guiding structure 138 is S-shaped. With this design of the anti-light-guiding structure 138, the propagation of infrared signals in an undesired direction may be reduced, so that the infrared light from the infrared transmitter 133 may only propagate to the infrared receiver 134 through the optical path shown by the arrow in Figure 9, thus facilitating the operator to accurately determine whether the clamping valve 31 has been mounted in place.

With this design, it is possible to form the infrared light guide 135 as a one-piece component with the same material in, for example, an integrated manner while making light propagate only through the light guiding column 136 in the infrared light guide 135. This reduces the production cost and manufacturing difficulty of the infrared light guide.

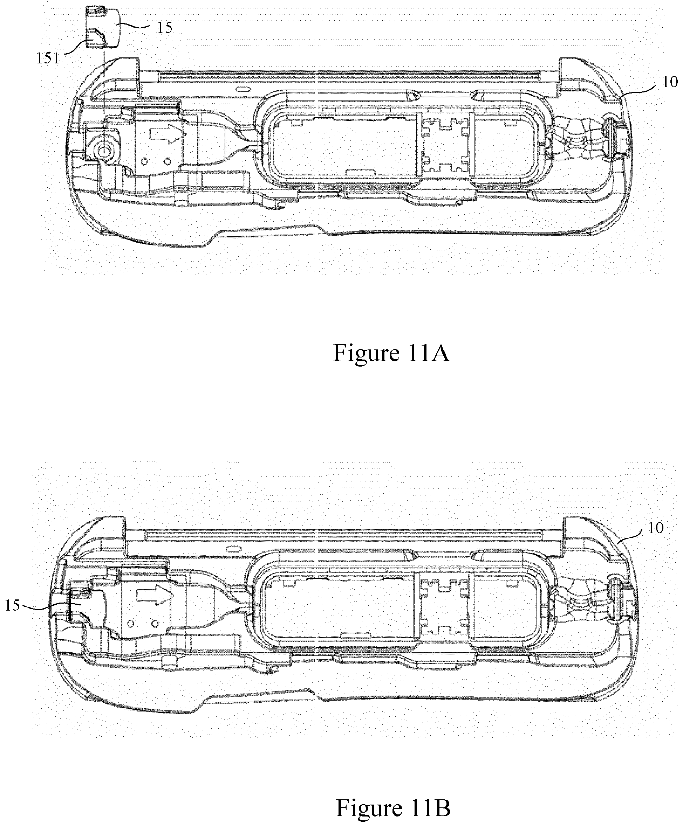

In addition, in order to ensure that the infusion line 20 is fixed in place in the body 10, in addition to the above-mentioned components for limiting the infusion line 20, the body 10 further includes a fixing piece 15 for fixing and limiting the infusion line 20. Figures 11A and 11B schematically show the fixing piece 15 and the body 10 of the infusion device 1 in a separated and connected state. The fixing piece 15 is arranged outside the liquid stopper mechanism 13, and includes elastic legs 151 on both sides of the fixing piece 15, and the bottom part of the fixing piece 15 is configured to have a shape structure matching with the corresponding part of the body 10 for connecting the fixing piece 15. In the process of connecting the fixing piece 15 to the body 10, the fixing piece 15 is connected to the body 10 by hot melt connection instead of traditional screw connection, which improves the waterproof performance of the joint, and reduces the number of connecting parts in the body 10, thereby reducing the weight of the body 10 and reducing the production cost.

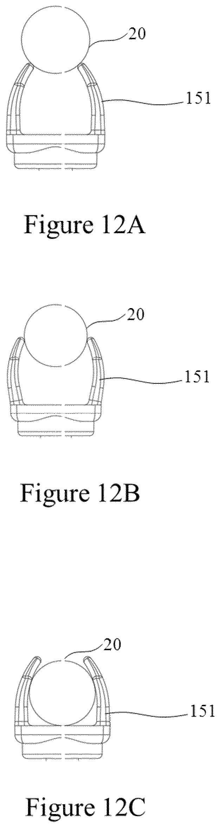

As shown in Figures 12A to 12C, in the process of snapping the infusion line 20 into the fixing piece 15, the elastic leg 151 may be elastically deformed to clamp and fix the infusion line in the fixing piece 15, which ensures that the infusion line is fixed in place in the body 10 and facilitates the operator to determine that the infusion line has been snapped in place in the fixing piece 15.

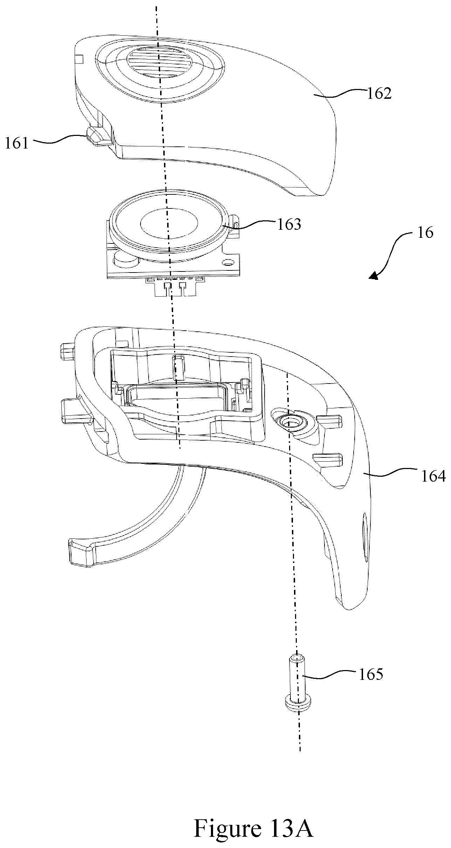

In addition, referring back to Figure 1, the infusion device 1 further includes a loudspeaker assembly 16 for giving an alarm to the operator when abnormal infusion conditions are detected. Figure 13A schematically shows an exploded perspective view of the loudspeaker assembly 16. As shown in the Figure, the loudspeaker assembly 16 includes: a battery door cover 162, where the battery door cover 162 is located at the outermost part of the loudspeaker assembly 16 and is used to close the loudspeaker assembly 16, and the battery door cover 162 is provided with two connecting protrusions 161 protruding and extending at one side of the battery door cover 162; loudspeaker 163; a battery door base 164, the battery door base 164 is used to receive components such as a loudspeaker 163, a battery door cover 162, and a battery not shown, and the battery door base 164 is configured to have a receiving recess for receiving the connecting protrusion 161; and a connecting piece 165, the connecting piece 165 is used to extend through the battery door cover 162 and the battery door base 164 to fixedly connect the two.

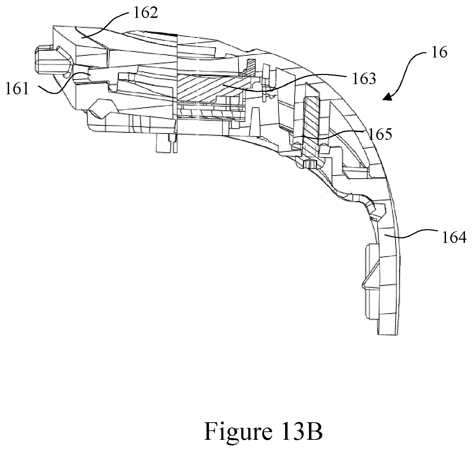

As shown in Figure 13B, in the assembled state of the loudspeaker assembly 16, the battery door cover 162 is fixedly connected to the battery door base 164 by means of two connecting protrusions 161 and connecting pieces 165 located at the sides of the battery door cover 162, and the loudspeaker 163 is also fixedly sandwiched between the battery door cover 162 and the battery door base 164. As a result, the loudspeaker assembly 16 is assembled under the condition that only one connecting piece (that is, the connecting piece 165 here) is required. This further reduces the number of connecting parts in the infusion device, thereby saving production costs and reducing assembly and manufacturing difficulties. It should be pointed out that in the illustrated embodiment, the connecting piece 165 is in the form of a bolt connecting member, but the connecting piece 165 may also be in the form of other connecting members suitable for connecting the battery door cover 162 and the battery door base 164.

As mentioned above, this application discloses some embodiments and refers to some possible alternatives, and these mentioned technical solutions are all within the protection scope of this application. In addition, certain obvious modifications conceived by those of ordinary skill in the art shall also fall within the protection scope of the appended claims.

While the present application has been described with reference to the exemplary embodiment, it will be appreciated that the present application is not limited to the specific embodiments/examples described and illustrated in detail herein. The person skilled in the art can make various variants to the exemplary embodiment without departing from the scope defined by the claims.

## Claims

1. An infusion device (1), the infusion device (1) comprising:
a body (10), the body (10) is configured to be able to receive an infusion line (20) and comprises a pumping mechanism (11), wherein the pumping mechanism (11) is configured to enable medical fluid to flow through the infusion line (20) received in the body (10);
a pump door assembly (30), wherein the pump door assembly (30) is configured to be connected to the body (10) for closing the body (10) , and is configured to fix the infusion line (20) in place in the body (10), the pump door assembly (30) comprises a buckle part (32), the buckle part (32) comprises: buckle protrusions (33) provided at both ends of the buckle part (32) along a longitudinal direction of the buckle part (32), and top surfaces of the buckle protrusions (33) are shaped to be suitable for receiving the infusion line (20); a groove (34), the groove (34) being located at an intermediate position between the buckle protrusions (33) and configured to be recessed in a height direction of the buckle part (32) to be lower than the buckle protrusions (33),
wherein
the body (10) further includes two receiving parts (14) the receiving parts are configured to cooperate with the buckle part (32) of the pump door assembly 30 to receive the infusion line (20) mounted in the body (10) and limit the infusion line (20), wherein
the body (10) of the infusion device (1) further comprises bubble sensors (12), the bubble sensors (12) are arranged directly below the receiving parts (14) at both sides of the infusion line (20) when the infusion line is received in the body (10), **characterized in that** the buckle part is configured such that
during the process that the infusion line (20) is pressed down by the buckle protrusions (33), an upward arched deformation generated by the infusion line (20) is accommodated in the groove (34) of the buckle part (32).

2. The infusion device (1) according to claim 1, **characterized in that**, the buckle part (32) further comprises transition recesses (35) configured to extend between the groove (34) and the buckle protrusions (33) from both sides of both longitudinal ends of the groove (34).

3. The infusion device (1) according to claim 1, **characterized in that**, a distance between the bubble sensors (12) is smaller than the diameter of the infusion line (20).

4. The infusion device (1) according to claim 3, **characterized in that**, the body (10) comprises a liquid stopper mechanism (13), the liquid stopper mechanism (13) comprises: a clamping valve (131) which can be deformed between a clamping position and a release position, in the clamping position, the clamping valve (131) presses the infusion line (20) passing through the clamping valve (131) to block the flow of medical fluid in the infusion line (20), in the release position, the clamping valve (131) allows the flow of medical fluid through the infusion line (20) , wherein the contour of the upper surface of the clamping valve (131) is a smoothly curved curve; a lever element (132), the level element (132) can be operated to act on the upper surface of the clamping valve (131) for switching the clamping valve (131) between the clamping position and the release position.

5. The infusion device (1) according to claim 4, **characterized in that**, the liquid stopper mechanism (13) comprises an infrared recognition assembly, and the infrared recognition assembly comprises: an infrared transmitter (133); an infrared receiver (134); and an infrared light guide (135), the infrared light guide (135) comprises: two light guide posts (136), the two light guide posts (136) are respectively positioned directly above the infrared transmitter (133) and the infrared receiver (134); and a connecting part (137), the connecting part (137) connects the two light guide posts (136).

6. The infusion device (1) according to claim 5, **characterized in that**, the connecting part (137) further comprises an anti-light-guiding structure (138) , and the anti-light-guiding structure (138) is configured as two anti-light-guiding grooves formed on two opposite surfaces of the connecting part (137), and the anti-light-guiding grooves extend along a width direction of the connecting part (137) and are staggered with each other, so that the side profile of the anti-light-guiding structure (138) is S-shaped.

7. The infusion device (1) according to claim 6, **characterized in that**, the infrared light guide (135) is a single-piece component formed by integral molding.

8. The infusion device (1) according to claim 1 or 2, **characterized in that**, the body (10) further comprises a fixing piece (15) for receiving the infusion line (20), and the fixing piece (15) comprises elastic legs (151) at two sides of the fixing piece (15), and when the infusion line (20) is received in the fixing piece (15), the elastic legs (151) are elastically deformed so that the infusion line (20) is clamped in the fixing piece (15).

9. The infusion device (1) according to claim 8, **characterized in that**, the bottom part of the fixing piece (15) is configured to have a shape structure matching with the corresponding part of the body (10) for connecting the fixing piece (15), and the fixing piece (15) is connected to the body (10) in a hot melt connection manner.

## Patentansprüche

1. Infusionsvorrichtung (1), wobei die Infusionsvorrichtung (1) Folgendes umfasst:
einen Körper (10), wobei der Körper (10) dazu konfiguriert ist, eine Infusionsleitung (20) aufnehmen zu können und einen Pumpmechanismus (11) umfasst, wobei der Pumpmechanismus (11) dazu konfiguriert ist, zu ermöglichen, dass medizinisches Fluid durch die in dem Körper (10) aufgenommene Infusionsleitung (20) fließt;
eine Pumpentürbaugruppe (30), wobei die Pumpentürbaugruppe (30) dazu konfiguriert ist, mit dem Körper (10) verbunden zu werden, um den Körper (10) zu schließen, und dazu konfiguriert ist, die Infusionsleitung (20) in dem Körper (10) an ihrem Platz zu fixieren, wobei die Pumpentürbaugruppe (30) ein Schnallenteil (32) umfasst, wobei das Schnallenteil (32) Folgendes umfasst: Schnallenvorsprünge (33), die an beiden Enden des Schnallenteils (32) entlang einer Längsrichtung des Schnallenteils (32) vorgesehen sind, und wobei obere Flächen der Schnallenvorsprünge (33) geformt sind, um zum Aufnehmen der Infusionsleitung (20) geeignet zu sein; eine Rille (34), wobei sich die Rille (34) an einer Zwischenposition zwischen den Schnallenvorsprüngen (33) befindet und dazu konfiguriert ist, in einer Höhenrichtung des Schnallenteils (32) ausgespart zu sein, um niedriger als die Schnallenvorsprünge (33) zu sein,
wobei
der Körper (10) ferner zwei Aufnahmeteile (14) enthält, wobei die Aufnahmeteile dazu konfiguriert sind, mit dem Schnallenteil (32) der Pumpentürbaugruppe 30 zusammenzuwirken, um die in dem Körper (10) montierte Infusionsleitung (20) aufzunehmen und die Infusionsleitung (20) zu begrenzen, wobei
der Körper (10) der Infusionsvorrichtung (1) ferner Blasensensoren (12) umfasst, wobei die Blasensensoren (12) direkt unterhalb der Aufnahmeteile (14) an beiden Seiten der Infusionsleitung (20) angeordnet sind, wenn die Infusionsleitung in den Körper (10) aufgenommen wird, **dadurch gekennzeichnet, dass** das Schnallenteil dazu konfiguriert ist, dass während des Prozesses, bei dem die Infusionsleitung (20) durch die Schnallenvorsprünge (33) nach unten gedrückt wird, eine von der Infusionsleitung (20) generierte, nach oben gewölbte Verformung in der Rille (34) des Schnallenteils (32) untergebracht wird.

2. Infusionsvorrichtung (1) nach Anspruch 1, **dadurch gekennzeichnet, dass** der Schnallenteil (32) ferner Übergangsaussparungen (35) umfasst, die dazu konfiguriert sind, sich zwischen der Rille (34) und den Schnallenvorsprüngen (33) von beiden Seiten der beiden Längsenden der Rille (34) zu erstrecken.

3. Infusionsvorrichtung (1) nach Anspruch 1, **dadurch gekennzeichnet, dass** ein Abstand zwischen den Blasensensoren (12) kleiner ist als der Durchmesser der Infusionsleitung (20).

4. Infusionsvorrichtung (1) nach Anspruch 3, **dadurch gekennzeichnet, dass** der Körper (10) einen Flüssigkeitsstoppmechanismus (13) umfasst, wobei der Flüssigkeitsstoppmechanismus (13) Folgendes umfasst: ein Klemmventil (131), das zwischen einer Klemmposition und einer Freigabeposition verformt werden kann, wobei das Klemmventil (131) in der Klemmposition auf die durch das Klemmventil (131) verlaufende Infusionsleitung (20) drückt, um den Fluss des medizinischen Fluids in der Infusionsleitung (20) zu blockieren, wobei das Klemmventil (131) in der Freigabeposition den Fluss des medizinischen Fluids durch die Infusionsleitung (20) zulässt, wobei die Kontur der oberen Fläche des Klemmventils (131) eine sanft gekrümmte Kurve ist; ein Hebelelement (132), wobei das Hebelelement (132) betreibbar ist, um auf die obere Fläche des Klemmventils (131) einzuwirken, um das Klemmventil (131) zwischen der Klemmposition und der Freigabeposition umzuschalten.

5. Infusionsvorrichtung (1) nach Anspruch 4, **dadurch gekennzeichnet, dass** der Flüssigkeitsstoppmechanismus (13) eine Infraroterkennungsbaugruppe umfasst, und die Infraroterkennungsbaugruppe Folgendes umfasst: einen Infrarotsender (133); einen Infrarotempfänger (134); und einen Infrarot-Lichtleiter (135), wobei der Infrarot-Lichtleiter (135) Folgendes umfasst: zwei Lichtleitpfosten (136), wobei die beiden Lichtleitpfosten (136) jeweils direkt über dem Infrarotsender (133) und dem Infrarotempfänger (134) angeordnet sind; und ein Verbindungsteil (137), wobei das Verbindungsteil (137) die beiden Lichtleitpfosten (136) miteinander verbindet.

6. Infusionsvorrichtung (1) nach Anspruch 5, **dadurch gekennzeichnet, dass** das Verbindungsteil (137) ferner eine Antilichtführungsstruktur (138) umfasst, und die Antilichtführungsstruktur (138) als zwei Antilichtführungsrillen konfiguriert ist, die auf zwei gegenüberliegenden Oberflächen des Verbindungsteils (137) ausgebildet sind, und die Antilichtführungsrillen sich entlang einer Breitenrichtung des Verbindungsteils (137) erstrecken und zueinander versetzt sind, so dass das Seitenprofil der Antilichtführungsstruktur (138) S-förmig ist.

7. Infusionsvorrichtung (1) nach Anspruch 6, **dadurch gekennzeichnet, dass** der Infrarot-Lichtleiter (135) eine einstückige Komponente ist, die durch integrales Gießen gebildet wird.

8. Infusionsvorrichtung (1) nach Anspruch 1 oder 2, **dadurch gekennzeichnet, dass** der Körper (10) ferner ein Fixierstück (15) zum Aufnehmen der Infusionsleitung (20) umfasst, und das Fixierstück (15) elastische Schenkel (151) an zwei Seiten des Fixierstücks (15) umfasst, und wenn die Infusionsleitung (20) in dem Fixierstück (15) aufgenommen wird, die elastischen Schenkel (151) elastisch verformt werden, so dass die Infusionsleitung (20) in dem Fixierstück (15) festgeklemmt wird.

9. Infusionsvorrichtung (1) nach Anspruch 8, **dadurch gekennzeichnet, dass** der untere Teil des Fixierstücks (15) dazu konfiguriert ist, eine Formstruktur aufzuweisen, die mit dem entsprechenden Teil des Körpers (10) zum Verbinden des Fixierstücks (15) übereinstimmt, und dass das Fixierstück (15) mit dem Körper (10) in einer Heißschmelzverbindung verbunden ist.

## Revendications

1. Dispositif de perfusion (1), le dispositif de perfusion (1) comprenant :
un corps (10), le corps (10) est conçu pour pouvoir recevoir une ligne de perfusion (20) et comprend un mécanisme de pompage (11), dans lequel le mécanisme de pompage (11) est conçu pour permettre au fluide médical de s'écouler à travers la ligne de perfusion (20) reçue dans le corps (10) ;
un ensemble porte de pompe (30), dans lequel l'ensemble porte de pompe (30) est conçu pour être relié au corps (10) pour la fermeture du corps (10), et est conçu pour fixer la ligne de perfusion (20) en place dans le corps (10), l'ensemble porte de pompe (30) comprend une partie de bouclage (32), la partie de bouclage (32) comprend : des saillies de bouclage (33) fournies au niveau de deux extrémités de la partie de bouclage (32) le long d'une direction longitudinale de la partie de bouclage (32), et des surfaces de dessus des saillies de bouclage (33) sont conformées pour être appropriées à la réception de la ligne de perfusion (20) ; une rainure (34), la rainure (34) étant située à une position intermédiaire entre les saillies de bouclage (33) et conçue pour être évidée dans une direction de hauteur de la partie de bouclage (32) pour être plus basse que les saillies de bouclage (33),
dans lequel
le corps (10) comporte en outre deux parties de réception (14), les parties de réception étant conçues pour coopérer avec la partie de bouclage (32) de l'ensemble porte de pompe (30) pour recevoir la ligne de perfusion (20) montée dans le corps (10) et limiter la ligne de perfusion (20), dans lequel
le corps (10) du dispositif de perfusion (1) comprend en outre des capteurs de bulles (12), les capteurs de bulles (12) sont agencés directement sous les parties de réception (14) des deux côtés de la ligne de perfusion (20) lorsque la ligne de perfusion est reçue dans le corps (10), **caractérisé en ce que** la partie de bouclage est conçue de telle sorte que pendant le processus au cours duquel la ligne de perfusion (20) est pressée vers le bas par les saillies de bouclage (33), une déformation en arc de cercle vers le haut générée par la ligne de perfusion (20) est logée dans la rainure (34) de la partie de bouclage (32).

2. Dispositif de perfusion (1) selon la revendication 1, **caractérisé en ce que**, la partie de bouclage (32) comprend en outre des évidements de transition (35) conçus pour s'étendre entre la rainure (34) et les saillies de bouclage (33) depuis les deux côtés de deux extrémités longitudinales de la rainure (34).

3. Dispositif de perfusion (1) selon la revendication 1, **caractérisé en ce que**, une distance entre les capteurs de bulles (12) est inférieure au diamètre de la ligne de perfusion (20).

4. Dispositif de perfusion (1) selon la revendication 3, **caractérisé en ce que**, le corps (10) comprend un mécanisme d'arrêt de liquide (13), le mécanisme d'arrêt de liquide (13) comprend : une vanne de serrage (131) qui peut être déformée entre une position de serrage et une position de relâchement, dans la position de serrage, la vanne de serrage (131) presse la ligne de perfusion (20) passant à travers la vanne de serrage (131) pour bloquer l'écoulement de fluide médical dans la ligne de perfusion (20), dans la position de relâchement, la vanne de serrage (131) permet l'écoulement de fluide médical dans la ligne de perfusion (20), dans lequel le contour de la surface supérieure de la vanne de serrage (131) est une courbe à courbure douce ; un élément de levier (132), l'élément de levier (132) peut être actionné pour agir sur la surface supérieure de la vanne de serrage (131) pour la commutation de la vanne de serrage (131) entre la position de serrage et la position de relâchement.

5. Dispositif de perfusion (1) selon la revendication 4, **caractérisé en ce que**, le mécanisme d'arrêt de liquide (13) comprend un ensemble de reconnaissance infrarouge, et l'ensemble de reconnaissance infrarouge comprend : un émetteur infrarouge (133) ; un récepteur infrarouge (134) ; et un guide de lumière infrarouge (135), le guide de lumière infrarouge (135) comprend : deux montants de guidage de lumière (136), les deux montants de guidage de lumière (136) sont respectivement positionnés directement au-dessus de l'émetteur infrarouge (133) et du récepteur infrarouge (134) ; et une partie de liaison (137), la partie de liaison (137) relie les deux montants de guidage de lumière (136).

6. Dispositif de perfusion (1) selon la revendication 5, **caractérisé en ce que**, la partie de liaison (137) comprend en outre une structure anti-guidage de lumière (138), et la structure anti-guidage de lumière (138) est conçue comme deux rainures anti-guidage de lumière formées sur deux surfaces opposées de la partie de connexion (137), et les rainures anti-guidage de lumière s'étendent le long d'une direction de largeur de la partie de liaison (137) et sont décalées l'une par rapport à l'autre, de sorte que le profil latéral de la structure anti-guidage de lumière (138) est conformée en S.

7. Dispositif de perfusion (1) selon la revendication 6, **caractérisé en ce que**, le guide de lumière infrarouge (135) est un composant monobloc formé par moulage intégral.

8. Dispositif de perfusion (1) selon la revendication 1 ou 2, **caractérisé en ce que**, le corps (10) comprend en outre une pièce de fixation (15) pour la réception de la ligne de perfusion (20), et la pièce de fixation (15) comprend des pattes élastiques (151) des deux côtés de la pièce de fixation (15), et lorsque la ligne de perfusion (20) est reçue dans la pièce de fixation (15), les pattes élastiques (151) sont déformées élastiquement de sorte que la ligne de perfusion (20) est serrée dans la pièce de fixation (15).

9. Dispositif de perfusion (1) selon la revendication 8, **caractérisé en ce que**, la partie inférieure de la pièce de fixation (15) est conçue pour avoir une structure de conformation s'appariant avec la partie correspondante du corps (10) pour la liaison de la pièce de fixation (15), et la pièce de fixation (15) est reliée au corps (10) d'une manière de liaison par fusion à chaud.
